# EUROPEAN PATENT SPECIFICATION

(11) **EP 0 937 478 B1**
(45) Date of publication and mention of the grant of the patent: **27.08.2003**
(21) Application number: 98102918.4
(22) Date of filing: 19.02.1998
(51) Int. Cl.: A61M 13/00

(54) **Device and apparatus for intracavitary drug delivery during video-assisted surgery or other endoscopic procedures**
Vorrichtung und Gerät zur intrakavitären Wirkstofffreigabe während videounterstützter Chirurgie oder anderer endoskopischer Verfahren
Dispositif et appareil pour l'administration de medicament intracavité pendant une chirurgie assistée par vidéo ou autres interventions endoscopiques

(43) Date of publication of application: 25.08.1999
(73) Proprietor: Microflow Engineering SA, 2007 Neuchâtel (CH)
(72) Inventor: Hess, Joseph, 2022 Bevaix (CH); Reymond, Marc, 1222 Vésenaz (CH); Bo, Hu, 2034 Peseux (CH)
(74) Representative: Kügele, Bernhard

(56) References cited:
- DE-A- 19 510 710
- US-A- 3 091 239
- US-A- 4 998 527
- US-A- 5 399 159

## Description

This invention relates to a device and a system for intracavitary drug delivery, e.g. during video-assisted surgery or other therapeutic procedures.

In current surgical practice, minimally invasive, video-assisted techniques, are developing rapidly. These techniques allow to carry out most of the abdominal, gynaecological, urological or thoracic surgical procedures, or other organ resections, through access ports and under videoscopic control. Recently, further fields of applications have appeared, such as axillary node dissection or other procedures elsewhere in the human body. These techniques are not only applied to the human patient, but nowadays also to veterinary medicine.

There are several advantages related to minimally invasive procedures compared to traditional surgery procedures. Minimally invasive procedures have been advocated to allow earlier recovery, less pain, less cardiopulmonary complications, less postoperative adhesions and less postoperative immunological depression.

Particularly in laparoscopic surgery procedures, the use of a C02-pneumoperitoneum (or alternative medium such as helium or other gases) to expand the abdominal cavity is employed routinely. Such media are occasionally used in thoracoscopic surgery, or in retroperitoneal laparoscopic surgery. They allow for a good exposition of the complete cavity in which the surgeon will operate. This is not the case when using mechanical wall-elevating devices, which only permit a limited visualisation of the abdomen (e.g. upper right quadrant for cholecystectomy).

However, the use of Co2 to expand the abdominal cavity has been claimed to have several disadvantages.

Indeed, in laparoscopic procedures for severe peritonitis, an increased risk of dissemination of bacteria into the blood stream (septicaemia) has been observed.

Further, in operations for cancer, an increased number of port-site recurrences has been observed after minimal invasive procedures, sometimes also at the remote site of tumour extraction. Such port-site recurrences have been observed after curative (intention to cure) as well as palliative (procedures for symptomatic relief). In operations for cancer, recurrences at peritoneal wounds have also been observed after laparoscopy or thoracoscopy procedures.

These side effects could therefore prevent the further development of minimally invasive surgery in peritonitis and in cancer. However, it may be understood that minimally invasive techniques could be beneficial for the patient if such side effects could be prevented.

It may thus be understood that there is a need for improved loco-regional therapy in cancer, particularly to treat any residual disease after surgery has taken place. This is particularly necessary to treat metastases on peritoneum, pleura or pericardium, or to reduce further postoperative adhesions.

Furthermore, it is desirable to prevent bacterial transfection into the vascular or lymphatic system by intra-operative therapy of intra-abdominal or intrathoracic infections.

It is to be noted that the term "therapy" as used throughout the description includes the use of any drug or therapeutic agent during minimally invasive procedures.

Further, it has been found that the pneumoperitoneum can be used as a vector, i.e. a therapeutic agent for therapy, thus introducing the concept of therapeutic pneumoperitoneum.

It is therefore, an object of the present invention to overcome the known problems and to provide a proper device which is able to distribute drugs or other therapeutic agents within the abdominal cavity during minimally invasive, e.g. laparoscopic, surgery, that could prevent most of the above-mentioned side-effects and allow to extent the indications for minimal invasive surgery. In a similar way, such a proper device is able to distribute the therapy into the thoracic cavity or into any other natural or artificial body cavity.

The present invention thus relates generally to intracavitary drug delivery devices suitable for minimally invasive surgery procedures, and in particular to such a device comprising a micro-pump having a droplet spray device for delivering a drug to a patient using a gas as a transport medium of the drug substance as set out in present claim 1. The present invention also relates to a system suitable for minimally invasive surgery incorporating such a device as defined in present claim 4.

A device for introcavitory drug delivery as described in the preamble of claim 1 is disclosed in US-A-5399159.

The micro-pump of the present invention delivers the drug, which is in the form of a liquid substance, as a dispersion of atomised droplets thanks to the incorporated monodispersive spray device. Thanks to the use of such a micro-pump in the inventive device, it is possible to distribute microdroplets of any therapeutic agent into the gas used to create a pneumoperitoneum. The device is located between a gas insufflator and the body cavity where surgery is performed, allowing to aerosolise microdroplets of the therapeutic substance into the pneumoperitoneum.

In fact, as is generally known, the efficacy of a drug therapy treatment depends on the substance's activity, which depends on the composition, on the place of impact, i.e. the place at which it may carry out its activity, and on the dose repeatability, i.e. the fact that the volume of each dose ejected remains constant, maintaining the concentration of the drug constant with the gas flow.

However, if droplet size varies, it is almost impossible to determine the quantity and where exactly the droplets will arrive. It is thus most desirable to control the size of the droplets and the desired flow to obtain an efficient therapy.

Preferably, such a device is small in size to allow for its use in minimally invasive therapies.

Control means are further provided for regulating the flow-rate, concentration and amount of drug delivery according to the gas flow, in a closed loop control system. In fact, it is important to know precisely at each moment of the operation the amount of therapeutic agent that has been administrated to a patient.

Other features and advantages of the device according to the present invention will become more clear from reading the following description which is given solely by way of a non-limitative example thereby referring to the attached drawings in which :
- Figure 1 shows schematically a system according to the present invention suitable for a minimally invasive surgery procedure and incorporating a device according to the present invention,
- Figure 2 shows in more detail the device of figure 1 comprising a micro-pump and its spray device, and
- Figure 3 shows an example of a suitable spray device for use with the micro-pump of the device according to the present invention.

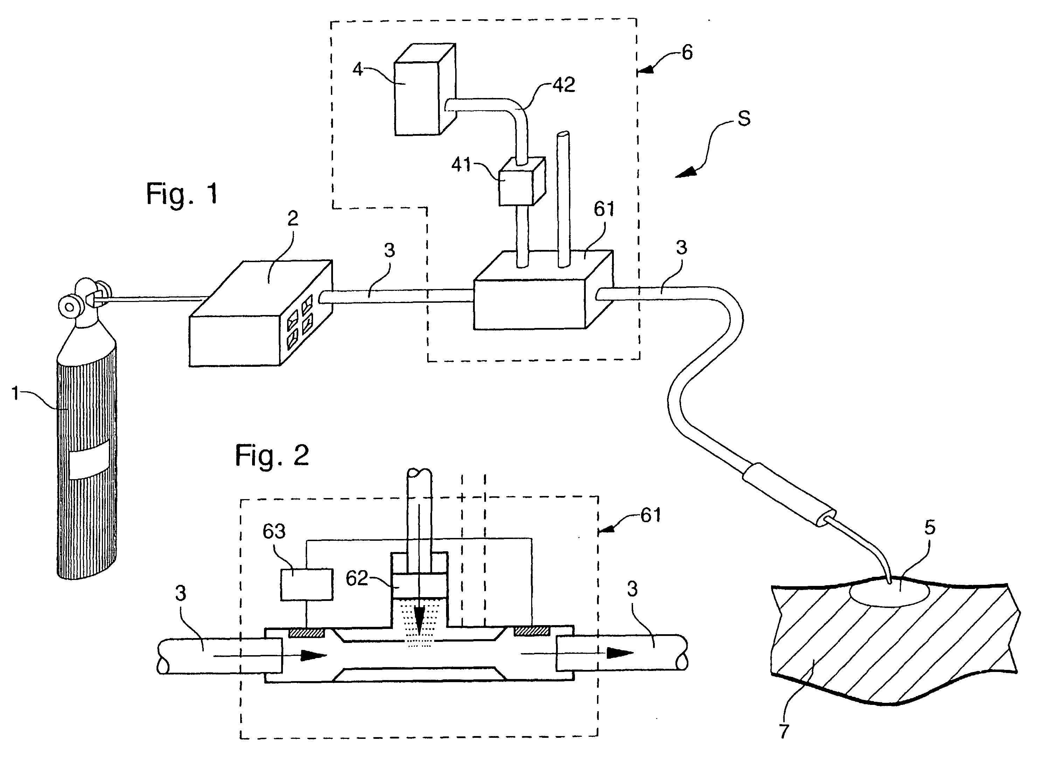

Figure 1 shows schematically the system and device according to the present invention for use in a minimally invasive surgery.

System S comprises a gas insufflator 2 having a gas input and output connection, this insufflator may be e.g. an electronic laparoflator such as is commercialised by the company Karl Storz, A gas supply 1 is connected to the input of insufflator 2 and contains e.g. CO2 or any other gas suitable for expanding a body cavity during surgery. An insufflation line, or tubing 3 is connected to the output of insufflator 2 and, in a known manner, for example via a trocar, to a cavity 5 created in a patient ? undergoing minimally invasive surgery so as to provide the gas at a certain flow rate. The gas insufflator may provide e.g. on a digital display (not referenced), in a known manner, information concerning the patient-insufflation line pressure and the gas flow rate thus allowing monitoring of these parameters.

Tubing 3 consists of two parts which are interconnected by way of a device 6. According to the present invention, device 6 comprises a micro-pump 61 which comprises a droplet spray device 62, and control means 63 for controlling e.g. the gas flow, as will be explained in more detail furtheron.

A drug reservoir 4 containing a therapeutic substance is connected to micro-pump 61 of device 6, e.g. via a tube 42. Preferably, a flow sensor 41 is provided between the reservoir 4 and micro-pump 61 for detecting the flow rate of the substance by measuring a differential pressure of the fluid stream along tube 42. To this effect, flow sensor 41 is provided with an input and an output pressure sensor. Such flow sensors are well known to the skilled person so that only a brief description will be given here. The flow sensor determines the difference between the two detected pressures to obtain a differential pressure which is proportional to the flow rate. Typically, such a pressure sensor comprises a thin micromachined diaphragm formed of silicon and a transducer overlying the diaphragm. The diaphragm may be obtained by etching the backside of a silicon wafer. Details of such a sensor may be found in European patent application EP 96 112 925.1 filed in the name of the present Applicant. Such a sensor may be advantageously used here, however, other flow sensors may of course be used too.

Additionally, a flush line, not referenced, may be provided which is a tube connected to micro-pump 61 allowing to clean the device 6 and tubing 3 or to evacuate any gas from tubing 3.

Figure 2 shows in more detail device 6 and its components.

Micro-pump 61 is formed of a venturi allowing the passage of the gas flow from insufflator 2 to cavity 5. Preferably, this micro-pump comprises input and output pressure sensors for determining the pressure flow of the gas towards the patient.

Micro-pump 61 is associated with a liquid droplet spray device 62 arranged so as to create a droplet spray of the therapeutic substance flowing from reservoir 4 into micro-pump 61. Thus this substance is vaporised into the gas flow so that it will be transported by this gas flow towards cavity 5 where it may carry out its therapeutic effect. Control means 63 are connected to micro-pump 61, to its sensors, and to spray device 62 to control the drug flow and concentration into tubing 3 leading to patient P.

The Applicant has used a spray device according to this description built directly into a trocar as commercially available.

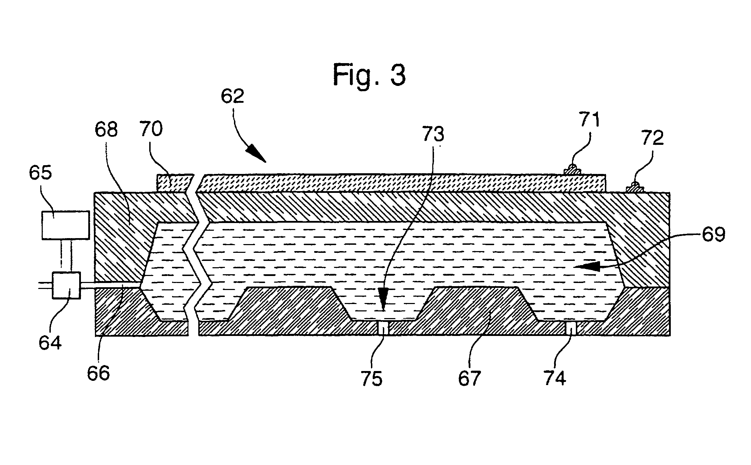

Figure 3 shows in detail such a preferred spray device to be used in the device according to the present invention.

Such a droplet spray device is known as such from European patent application no. 97 120 287.4 in the name of the present Applicant.

This spray device will now be described briefly with reference to figure 3 which corresponds to figure 2 of the above mentioned European patent application.

Spray device 62 consists in this example of a housing formed of a superposition of a top substrate 68 and a bottom substrate 67 into which a space 69 is etched which contains the liquid substance. Space 69 contains a unit dose, such as 10 to 30 µl or another suitable small amount of drug substance. The substrates 67 and 68 are attached to each other, for example by anodic or fusion bonding, so as to enclose space 69. Spray device 62 further comprises a piezoelectric element 70 attached to the exterior top surface of top substrate 68 to cause vibration of the substance in space 69. This element may be glued to or deposited on this top surface and is excited in an appropriate manner e.g. using electrodes 71, 72 and a frequency oscillator as described in the above referenced document.

Outlet nozzles 74 are formed in the nozzle body of the bottom substrate 67, so that the excited substance 4 may leave spray device 62 as a droplet spray. To this effect, this bottom substrate 67 is micromachined, for example in a well known anisotropic etching manner, at several places to obtain tapered, pyramid-shaped cavities 73 which are for example about 200 to 400 µm deep. Each cavity 73 then has a bottom surface having a side length of about 100 - 200 µm. Within this bottom surface of each cavity 73 at least one output channel 75 is provided to connect cavity 73 to the exterior of bottom substrate 67. To this effect, at the outer end of each output channel 15 at least one output nozzle 74 is provided through which the excited liquid substance is ejected as a droplet spray. Dimensions as well as the number of nozzles may of course be readily modified depending on the amount of drug and on the corresponding droplet size to be ejected as will be explained in more detail here after. However, it should be noted that the length of this output channel 75 should not be too long to avoid a large pressure drop across this channel resulting in a change of the droplet size as ejected.

It has been observed that the droplets size is inversely proportional to the excitation frequency as of a particular frequency so that piezoelectric element 70 preferably vibrates at that particular frequency which may be for example around 470 kHz.

A hydrophilic coating, such as an amorphous material such as SiO2, may further be provided to provide a protective layer around the inside walls of space 69 and/or the cavities to avoid any contamination of the material of these walls with the substance. This may be carried out as described in the document EP-A-0 641 934.

Thus, liquid spray device 62 creates droplets of this substance by atomising the liquid substance and releasing them, via the housing of micro-pump 61, into tubing 3 connected to insufflator 2 and to cavity 5.

However, it should be noted that the invention is not limited to this specific spray device. In fact, a spray device of the so-called side-shooter type may also be used. Such a device comprises output nozzles located at the side wall of the main housing. Also, it is possible to replace the output nozzles by one or more capillary outlets. Such an arrangement allows for a facilitated entering of the substance into the gas flow.

The liquid substance may enter spray device 62 by way of e.g. a very low pressure or capillary action, for example by way of at least one supply tube or needle 66 through which the liquid substance may flow from reservoir 4 into spray device 62. However, this filling may also be piston or plunger activated or even performed by way of a pump. A micro-valve 64 may be provided to isolate the reservoir from spray device 62. This valve 64 may be external to the spray device, but it can also be integrated into the substrate. Preferably, it is controlled by flow measurement means 65 which are provided and which may be incorporated in spray device 62 thus allowing precise dosage in conjunction with the valve.

The reservoir 4 can be realised in a variety of forms so as to contain from several microliters to several millilitres. Advantageously, this reservoir can even be incorporated in micro-pump 61 or in spray device 62 as is described in the above mentioned European patent application.

In a preferred embodiment, flow measurement means 65 are realised in the way of a differential pressure sensor allowing a more complete supervision of the spray device by not only measuring the flow, but also by detecting an empty reservoir 4 as well as a possible occlusion of the output nozzles of spray device 62.

Preferably, a temperature influence compensation of the frequency oscillator may be provided. Advantageously, means 65 may be adapted to perform this compensation in a manner which may be readily conceived by a skilled person.

Thanks to such a spray device, virtually mono-dispersive droplets are ejected which allow for a precise calculation of the amount of drug which will enter the gas flow in the tubing. As the top surface of each cavity 73 is much larger than the actual nozzle surface, it is thus possible to provide several outlet nozzles 74 on each cavity surface in order to eject more droplets simultaneously, and thus a larger amount of drug.

As mentioned here above, the device according to the present invention further comprises control means 63 for controlling, amongst others, the amount of droplets to be ejected. These control means advantageously further comprise a gas flow sensor located in the tubing interface. In a known manner, this gas flow sensor has input and output pressure sensors for determining the differential pressure of the gas which is indicative of the flow rate. In the present invention, the gas flow sensor is used in conjunction with drug flow measurement means 65 to effectively measure and control drug flow and concentration during the operation.

Thus, it is clear that a skilled person may determine, for a given gas flow rate, the total amount and concentration of drug to be released. Indeed, the droplet size may be determined by the specific structure, and as the droplets are highly mono-dispersive, the total amount released may be determined.

Advantageously, the micro-pump 61 and its spray device 62 may be used in a highly reliable drug delivery system which comprises to this effect at least two spray devices. As the flow measurement means 65 allow to detect plugging or blocking of nozzles and/or an empty reservoir as is mentioned here above, it is thus also possible to monitor the operation of the spray device. The drug delivery system may thus, if a spray device is considered to be inoperative, switch to another spray device to maintain the intended drug flow. Appropriate control means may be provided to this effect, as such means may be conceived readily by a skilled person, they will not be described in detail here. Thanks to this redundancy of spray devices, a continuous reliable operation of the drug delivery system is obtained.

In a particular aspect, the invention allows the distribution of therapeutic agents with a low viscosity and a pH value around the physiological value. In particular, by using the above-mentioned invention, aerosolisation of microdroplets such as povidone-iodine solution, of differentiating or immunomodulating agents such as butyrate and of adhesion-preventing agents such as taurolidin (Taurolin®), Sepracoat® is possible.

The invention includes the use of the above described device 6 with its micro-pump for any intracavitary drug delivery in the human body or in the animal body (e. g. in veterinary medicine). It also includes the use of various kinds of micro-pumps, a separation of the venturi and micropumps or a combination of different micro-pumps for intracavitary delivery of drugs during minimally invasive procedures, e. g. when a higher volume, an extreme pH or a higher viscosity are mandatory for effective drug delivery. Also, the Applicant has devised a solution for incorporating the micropump directly into a tracor, used to enter cavity 5 and provide the path for the gas and the vaporised medication.

While the invention relates principally to laparoscopy, it is applicable to any other intracavitary procedure such as thoracoscopy, retroperitoneoscopy, axilloscopy, properitoneoscopy and cervicoscopy for example, if a gas is used as transport medium for drug delivery, the invention also applies to any other endoscopic procedure such as oesogastroduodenoscopy, rectosignoidoscopy, colonoscopy, etc. and other intensive care therapy administration.

A preferred use of the device according to the present invention for intracavitary drug delivery is the application of any proteoglycans (such as heparin, hylouronates, taurolidine, etc.) to prevent the formation of adhesions. This not only prevents postoperative symptoms such as occlusions, but may also reduces the rate of trocar site implantations, as described by J. Ordemann, Langenbecks Arch Chir I (Forumband 1997), 271. Application of hyluronate also alters cell-matrix adhesion in vitro and could reduce postoperative tumour growth, as described by C. Nduka, 10th Tripartite Meeting of the Surgical Research Society, Nottingham, 9, 10 & 11 July 1997.

Another way of using the inventive device consists in the use of a bactericidal agent such as povidone-iodine solution or any other soluble disinfectant as the therapeutic substance to reduce intra-abdominal or intrathoracal infectious complications, as described by W.F. Sindelar, J Hosp Infection (1985) S6 : 103-114.

The invention is useful in its own right for improved cancer therapy. Conveniently, laparoscopy is used for palliative or curative treatment of solid tumours such as colorectal carcinoma. Intrapertoneal delivery of cytocidal drugs such as 5-fluorouracile has been shown to be effective in conventional, open surgery (P.H. Sugerbaker), Dis Colon Rectum (1993) 36 : 323-329) and using cytocidal agents in the pneumoperitoneum could reduce the postoperative local recurrence rate.

Using the pneumoperitoneum as a therapeutic agent for immunomodulation of cancer cells using e.g. the differentiation agent butyrate (P. Perrin, Gastroenterology (1994) 107 : 1697-1708) could be promising in combination with a preserved immunity after laparoscopy as compared to conventional, open surgery. A better effect could be even obtained using combined hyperthermia (A. Menoret, J Immunol (1995) 155 : 740-747.

By extension, this device according to the present invention allows the distribution of any other therapy with low viscosity and pH between 6 and 9 when gas is insufflated into any natural or artificial body cavity.

Thanks to the specific structure of the dispersion or outlet nozzle of spray device 62, it is possible to obtain a virtually mono-dispersive droplet size and thanks to the control means incorporated into the inventive device it is also possible to provide closed loop control allowing to conclude with precision on the delivered dose and its concentration used for therapeutic pneumoperitoneum such as described above. Thus, an efficient therapy may be performed.

Having described the preferred embodiments of this invention, it will now be apparent to one of skill in the art that other embodiments incorporating its concept may be used. It is felt, therefore, that this invention should not be limited to the disclosed embodiment, but rather should be limited only by the scope of the appended claims.

## Claims

1. Device (6) for intracavitary drug delivery for distributing a therapeutic substance into a body cavity (5) of a patient (P), said device being arranged between said cavity (5) and a gas insufflator (2) including a tubing (3) connecting said insufflator to said cavity via said device and providing a gas to said cavity so as to expand said cavity, wherein said device comprises
- a pump (61) having control means (63) for controlling the gas flow through said pump and having a droplet spray device (62) for ejecting droplets of said therapeutic substance into said gas, and
- a drug reservoir (4) for providing said therapeutic substance to said spray device (62),
such that said gas becomes a transport medium for said drug delivery, **characterised in that**
said pump is a micro-pump, **in that**
said device further comprises a therapeutic substance flow rate sensor (41) for detecting the flow rate of the substance by measuring a differential pressure of the fluid stream, **in that**
said micro-pump further comprises a micro-valve (64) controlled by flow measuring means (65) to control the therapeutic substance flow, and **in that**
said control means (63) comprises a gas flow sensor,
such that said device controls the therapeutic substance flow thus controlling the amount of therapeutic substance ejected by said droplet spray device and the gas-therapeutic substance concentration of the drug delivery across the tubing (3).

2. Device according to claim 1, wherein said spray device (62) ejects monodispersive droplets.

3. Device according to claim 1 or 2, wherein said reservoir (4) is incorporated in said micro-pump (61),

4. Apparatus for intracavitary drug delivery for distributing a therapeutic substance into a body cavity (5) of a patient (P) and comprising:
- a gas insufflator (2),
- a tubing (3) connecting said insufflator (2) to said cavity (5) and providing a gas to said cavity so as to expand said cavity, **characterised in that** said system further comprises:
- a device (6) according to anyone of the preceding claims connected to said tubing (3) and between said cavity (5) and said gas insufflator (2) such that said gas becomes a transport medium for said drug delivery.

## Patentansprüche

1. Vorrichtung (6) zur intrakavitären Wirkstofffreigabe für die Abgabe einer therapeutischen Substanz in den Körperhohlraum (5) eines Patienten (P), bei Anordnung der Vorrichtung zwischen dem Hohlraum (5) und einem Gasinsufflator (2), der über einen Schlauch (3) verfügt, der den Insufflator mit dem Hohlraum über die Vorrichtung verbindet und ein Gas in den Hohlraum einleitet, sodass der Hohlraum erweitert wird, wobei die Vorrichtung umfasst:
- eine Pumpe (61) mit Steuerelementen (63) zur Steuerung des Gasflusses durch die Pumpe, die über einen Tröpfchen-Vernebler (62) zur Abgabe von Tröpfchen der therapeutischen Substanz an das Gas verfügt, und
- einen Wirkstoffbehälter (4) zur Abgabe der therapeutischen Substanz an den Vernebler (62),
sodass das Gas zu einem Transportmedium für die Wirkstofffreigabe wird, **dadurch gekennzeichnet, dass**
die Pumpe eine Mikropumpe ist, und dass
die Vorrichtung ferner einen Sensor (41) für die Flussrate der therapeutischen Substanz zur Ermittlung der Flussrate der Substanz durch Messung eines Differenzialdrucks des Flussstroms umfasst, und dass
die Mikropumpe ferner ein Mikroventil (64) umfasst, das durch Flussmesselemente (65) gesteuert wird, um den Fluss der therapeutischen Substanz zu steuern, und dass
die Steuerelemente (63) einen Gasflusssensor umfassen,
sodass die Vorrichtung den Fluss der therapeutischen Substanz steuert und derart die Menge an therapeutischer Substanz, die vom Vernebler ausgestossen wird, sowie die Konzentration der gasförmigen therapeutischen Substanz bei der Wirkstofffreigabe über den Schlauch (3) steuert.

2. Vorrichtung gemäß Anspruch 1, wobei der Vernebler (62) monodisperse Tröpfchen ausstösst.

3. Vorrichtung gemäß Anspruch 1 oder 2, wobei der Behälter (4) in die Mikropumpe (61) integriert ist.

4. Gerät zur intrakavitären Wirkstofffreigabe für die Abgabe einer therapeutischen Substanz in den Körperhohlraum (5) eines Patienten (P), das umfasst:
- einen Gasinsufflator (2),
- einen Schlauch (3), der den Insufflator (2) mit dem Hohlraum (5) verbindet und ein Gas in den Hohlraum einleitet, sodass der Hohlraum erweitert wird, **dadurch gekennzeichnet, dass** das Gerät ferner umfasst:
- eine Vorrichtung (6) nach einem der vorhergehenden Ansprüche, verbunden mit dem Schlauch (3) und zwischen dem Hohlraum (5) und dem Gasinsufflator (2) angeordnet, sodass das Gas zu einem Transportmedium für die Wirkstofffreigabe wird.

## Revendications

1. Dispositif (6) destiné à une délivrance de médicament endocavitaire en vue de distribuer une substance thérapeutique dans une cavité du corps (5) d'un patient (P), ledit dispositif étant agencé entre ladite cavité (5) et un insufflateur de gaz (2) comprenant un tube (3) reliant ledit insufflateur à ladite cavité par l'intermédiaire dudit dispositif et fournissant un gaz à ladite cavité de façon à dilater ladite cavité, où ledit dispositif comprend
- une pompe (61) comportant des moyens de commande (63) destinés à commander le débit de gaz à travers ladite pompe et comportant un dispositif de pulvérisation de gouttelettes (62) destiné à éjecter des gouttelettes de ladite substance thérapeutique dans ledit gaz, et
- un réservoir de médicament (4) destiné à fournir ladite substance thérapeutique audit dispositif de pulvérisation (62),
de sorte que ledit gaz devient un milieu de transport pour ladite délivrance de médicament, **caractérisé en ce que**
ladite pompe est une micropompe, **en ce que**
ledit dispositif comprend en outre un capteur de débit de substance thérapeutique (41) destiné à détecter le débit de la substance en mesurant une pression différentielle du flux de fluide, **en ce que**
ladite micropompe comprend en outre une microvanne (64) commandée par des moyens de mesure de débit (65) pour commander le débit de la substance thérapeutique, et **en ce que**
lesdits moyens de commande (63) comprennent un capteur de débit de gaz,
de sorte que ledit dispositif commande le débit de substance thérapeutique en commandant ainsi la quantité de substance thérapeutique éjectée par ledit dispositif de pulvérisation de gouttelettes et la concentration de substance gazeuse thérapeutique d'administration de médicament à travers le tube (3).

2. Dispositif selon la revendication 1, dans lequel ledit dispositif de pulvérisation (62) éjecte des gouttelettes monodispersives.

3. Dispositif selon la revendication 1 ou 2, dans lequel ledit réservoir (4) est incorporé dans ladite micropompe (61).

4. Appareil destiné à une délivrance endocavitaire de médicament en vue de distribuer une substance thérapeutique dans une cavité du corps (5) d'un patient (P) et comprenant :
- un insufflateur de gaz (2),
- un tube (3) reliant ledit insufflateur (2) à ladite cavité (5) et fournissant un gaz à ladite cavité de façon à dilater ladite cavité, **caractérisé en ce que** ledit système comprend en outre :
- un dispositif (6) selon l'une quelconque des revendications précédentes relié audit tube (3) et entre ladite cavité (5) et ledit insufflateur de gaz (2) de sorte que ledit gaz devient un milieu de transport pour ladite délivrance de médicament.
